# EUROPÄISCHE PATENTSCHRIFT

(11) **EP 3 921 063 B1**
(45) Veröffentlichungstag und Bekanntmachung des Hinweises auf die Patenterteilung: **10.01.2024**
(21) Anmeldenummer: 20702317.7
(22) Anmeldetag: 04.02.2020
(51) Int. Cl.: B01D 53/04, A61M 16/00, A61M 16/10

(54) **ZWEISTUFIGES VERFAHREN DER RÜCKGEWINNUNG VON HALOGENIERTEN KOHLENWASSERSTOFFEN**
TWO-STEP PROCESS FOR THE RECOVERY OF HALOGENATED HYDROCARBONS
PROCÉDÉ EN DEUX ÉTAPES DE RÉCUPÉRATION DES HYDROCARBURES HALOGÉNÉS

(30) Priorität: 05.02.2019 EP 19155562
(43) Veröffentlichungstag der Anmeldung: 15.12.2021
(73) Patentinhaber: ZeoSys Medical GmbH, 14943 Luckenwalde (DE)
(72) Erfinder: FRIEDRICH, Thomas, 10367 Berlin (DE); EWERS, Christian, 14943 Luckenwalde (DE)
(74) Vertreter: Schulz Junghans Patentanwälte PartGmbB
(86) Internationale Anmeldenummer: PCT/EP2020/052713
(87) Internationale Veröffentlichungsnummer: WO 2020/161115

(56) Entgegenhaltungen:
- EP-A1- 2 926 897
- EP-A2- 0 284 227
- DE-A1-102006 027 127
- DE-A1-102007 048 892
- US-A- 3 332 854
- US-A1- 2016 023 157
- US-A1- 2016 096 793
- US-A1- 2017 056 610

## Beschreibung

### Hintergrund

Inhalationsanästhetika werden verabreicht, um an einem Patienten eine Narkose einzuleiten oder aufrechtzuerhalten. Die verabreichten Inhalationsanästhetika werden überwiegend über die Atemluft des Patienten wieder an die Umgebung abgegeben, weshalb selbige in Operationssälen kontinuierlich abgesaugt und nachteilig für die Umwelt über Dach entsorgt werden. Denn Inhalationsanästhetika aus der Klasse der Flurane bestehen aus halogenierten Kohlenwasserstoffen. Diese sind starke Treibhausgase und ozonschichtschädigend. Die Rückgewinnung von Inhalationsanästhetika aus der Atemluft von Patienten ist daher zum Schutz der Gesundheit von Krankenhauspersonal (in Bereichen ohne Absaugung), aus Umweltschutzgründen und auch aus ökonomischer Sicht unabdingbar.

Es sind bereits Vorrichtungen und Verfahren bekannt, mit denen Inhalationsanästhetika aus der Atemluft von Patienten an Filtermaterialien sowohl adsorbiert als auch wieder desorbiert werden können.

WO2007093640 beschreibt eine Filterpatrone, die zur Adsorption und Desorption von halogenierten Kohlenwasserstoffen eingerichtet ist.

EP2946826 beschreibt eine Filteranlage für ein Gebäude, insbesondere für ein Krankenhaus, die dazu eingerichtet ist, Narkosegase aus dem die Filteranlage durchströmenden Gasgemisch herauszufiltern.

EP2926897 offenbart eine Vorrichtung zur Rückgewinnung von Narkosegasen, insbesondere von halogenierten Kohlenwasserstoffen, die dazu eingerichtet ist, an Filtermaterialien adsorbierte halogenierte Kohlenwasserstoffe mittels Dampf zu desorbieren.

EP2237854 und WO2008/017566 beschreiben ein Verfahren und einen Filter zur Adsorption von halogenierten Kohlenwasserstoffen und deren anschließende Desorption aus dem Filter.

WO2007093640 beschreibt eine Filterpatrone, die zur Adsorption und Desorption von halogenierten Kohlenwasserstoffen eingerichtet ist.

DE 10 2006 027127 offenbart eine Anlage zur Rückgewinnung von halogenierten Kohlenwasserstoffen und Ethern, insbesondere Inhalationsanästhetika, durch Desorption von einem mikroporösen Adsorbens mittels eines Wasserdampfstroms.

US 2016/023157 beschreibt ein Verfahren zur Trennung von Kohlendioxid von einer Gasmischung, wobei die Gasmischung zunächst bei einem ersten Druck an einer Adsorptionseinheit adsorbiert und anschließend bei einem höheren zweiten Druck aus der Adsorptionseinheit ausgeleitet wird.

US 3 332 854 offenbart eine Apparatur zur Rückgewinnung von Lösungsmitteln durch Adsorption an Aktivkohle und anschließende Desorption durch einen Dampfstrom.

EP 0 284 227 beschreibt ein Verfahren zur Rückgewinnung halogenierter Kohlenwasserstoffe aus einem Gasstrom durch Adsorption an einem hydrophoben Molekularsieb-Adsorbens und anschließendes Durchströmen mit einem Spülgas.

DE 10 2007 048892 offenbart einen Narkosegaszwischenspeicher mit einem elektrisch beeinflussbaren Adsorptionsmaterial.

US 2017/056610 offenbart ein System zum Sammeln eines Anästhesiegases, welches das Anästhesiegas in einem Speichersystem adsorbiert.

US 2016/096793 beschreibt ein System und ein Verfahren zur Rückgewinnung von halogenierten Kohlenwasserstoffen aus einem Gasstrom, wobei die Kohlenwasserstoffe an einem Adsorbens mit einer Gitterstruktur adsorbiert und anschließend mit einem Spülgas von dem Adsorbens gelöst werden.

Um rückgewonnene Inhalationsanästhetika erneut zur Narkose von Patienten verwenden zu können, ist es notwendig, die Inhalationsanästhetika zu sterilisieren. Es sind aus dem Stand der Technik noch keine Verfahren und keine Vorrichtungen bekannt, die dazu ausgelegt sind, Inhalationsanästhetika in einem Prozess rückzugewinnen und zu sterilisieren.

### Aufgabe der Erfindung

Aufgabe der Erfindung ist es daher, ein Verfahren und eine Vorrichtung zur verbesserten Rückgewinndung von Inhalationsanästhetika bereitzustellen. Diese Aufgabe wird gelöst durch ein Verfahren zur Rückgewinnung von fluorhaltigen Inhalationsanästhetika nach Anspruch 1. Vorteilhafte Ausführungsformen des Verfahrens sind in den Ansprüchen 2 bis 8 dargestellt. Diese Aufgabe wird weiter durch eine Vorrichtung nach Anspruch 9 gelöst. Vorteilhafte Ausführungsformen der Vorrichtung sind in den Ansprüchen 10 bis 13. dargestellt.

### Definitionen

Der Begriff *Adsorbens* oder *Sorptionsmittel* bezieht sich im Kontext der vorliegenden Beschreibung auf ein Material, das Gase an seiner Oberfläche adsorbieren kann. *Adsorbens* und *Sorptionsmittel* werden in der vorliegenden Erfindung synonym verwendet.

Der Begriff *Sorbat* bezeichnet das Adsorbens mit den daran adsorbierten Gasen.

Der Begriff *Desorbat* bezeichnet die desorbierten Gase.

Der Begriff *Sorptiv* bezeichnet die zu adsorbierenden Gase.

Der Begriff *halogenierte Kohlenwasserstoffe* bezeichnet insbesondere fluorhaltige Inhalationsanästhetika.

### Detaillierte Beschreibung der Erfindung

Ein erster Aspekt der Erfindung betrifft ein Verfahren zur Rückgewinnung von halogenierten Kohlenwasserstoffen. In diesem Verfahren wird in einem Desorptionsschritt ein adsorbierte halogenierte Kohlenwasserstoffe umfassendes Adsorbens von einem im Wesentlichen aus trockenem Wasserdampf bestehenden Volumenstrom insbesondere bei erhöhter Temperatur durchströmt, (wobei insbesondere das adsorbierte halogenierte Kohlenwasserstoffe umfassende Adsorbens von Wasserdampf durchströmt wird). Dadurch werden die adsorbierten halogenierten Kohlenwasserstoffe vom Adsorbens desorbiert und in den Volumenstrom aufgenommen, wodurch ein halogenierte Kohlenwasserstoffe und Wasserdampf enthaltender sekundärer Volumenstrom entsteht. Dieser sekundäre Volumenstrom wird durch Kühlung in ein halogenierte Kohlenwasserstoffe und Wasser enthaltendes Kondensat überführt. Aus diesem Kondensat werden die halogenierten Kohlenwasserstoffe abgetrennt. Die Temperatur liegt bei diesem Schritt insbesondere bei 100°C oder darüber. Verschiedene Verfahrensvarianten sehen eine Durchführung dieses Schrittes insbesondere bei einer Temperatur von 100°C bis 150°C vor, weiter insbesondere bei einer Temperatur von 120°C bis 150°C.

Erfindungsgemäß geht dem Desorptionsschritt ein Sterilisationsschritt voran. Dabei wird insbesondere das Sorbat vor dem Durchströmen mit Wasserdampf einer heißen Wasserdampfatmosphäre ausgesetzt. Die Erfinder haben die Parameter dieses Schrittes ermittelt, welche notwendig und hinreichend sind, aus potentiell mit pathogenen Organismen kontaminierten Sorbaten wiedergewonnene Wirkstoffe so zu behandeln, dass sie den Vorschriften der Europäischen Arzneimittelbehörden und analoger Vorschriften anderer Länder entsprechen. Der Desorptionsschritt und der Sterilisationsschritt werden insbesondere zeitlich unmittelbar aufeinanderfolgend durchgeführt, um die Effektivität des Prozesses zu erhöhen. Jedoch ist es auch denkbar, eine Pause zwischen dem Desorptionsschritt und dem Sterilisationsschritt vorzusehen. Weiterhin können der Desorptionsschritt und der Sterilisationsschritt insbesondere in derselben Anlage oder in unterschiedlichen Anlagen durchgeführt werden. Dabei kann z.B. ein Desorptionsbehälter zunächst an eine Sterilisationsanlage gekoppelt werden und anschließend an eine Desorptionsanlage.

Erfindungsgemäß wird während des Sterilisationsschrittes das die adsorbierten halogenierten Kohlenwasserstoffe umfassende Adsorbens für mindestens 10 min, insbesondere für 10 min bis 60 min, bei einer Temperatur von mehr als 120°C, insbesondere bei einer Temperatur von 121°C bis 150°C, und bei einem Druck von 0,15 MPa bis 0,4 MPa, insbesondere bei einem Druck von 0,15 MPa bis 0,3 MPa, in Kontakt mit, insbesondere trockenem, Wasserdampf gebracht.

In einer Ausführungsform wird das die adsorbierten halogenierten Kohlenwasserstoffe umfassende Adsorbens für 20 min bis 40 min, insbesondere für ungefähr 30 min, bei einer Temperatur von 135°C bis 145°C, und einem Druck von 0,24 MPa bis 0,26 MPa in Kontakt mit, insbesondere trockenem, Wasserdampf gebracht.

Durch die Kombination der Parameter Sterilisationszeit, -temperatur und -druck werden Zustände geschaffen, bei denen eine vollständige Inaktivierung aller im Sorbat befindlichen Pathogene gewährleistet wird.

Gleichzeitig sind die oben aufgeführten Parameter Sterilisationszeit, -temperatur und -druck so gewählt, dass die adsorbierten halogenierten Kohlenwasserstoffe unter den herrschenden Bedingungen nicht zersetzt werden oder chemische Reaktionen eingehen, sodass die sterilisierten halogenierten Kohlenwasserstoffe nach der Desorption erneut verwendet werden können.

Gemäß einer Ausführungsform wird das Adsorbens während des Sterilisationsschrittes nicht von Wasserdampf durchströmt. Gemäß einer Ausführungsform wird das Adsorbens insbesondere einmal, beim Einleiten des Wasserdampfes, vom Wasserdampf durchströmt und bleibt dann in einer Dampfatmosphäre bei Überdruck. Insbesondere erst nach Abschließen des Sterilisationsschrittes wird das Adsorbens zur Desorption der halogenierten Kohlenwasserstoffe vom Wasserdampf durchströmt.

Das Aufbauen des Drucks im Sterilisationsschritt und das Durchströmen des Adsorbens mit Wasserdampf beim Desorptionsschritt kann z.B. durch Öffnen und Schließen eines Ventils eines Desorptionsbehälters gesteuert werden. Alternativ dazu können auch andere manuelle Maßnahmen vorgesehen sein, um die Schritte des Verfahrens durchzuführen. So könnte z.B. ein Desorptionsbehälter nacheinander mit Leitungen einer Sterilisationsanlage und einer Desorptionsanlage manuell verbunden werden, wobei mittels der Sterilisationsanlage der Druck in dem Behälter aufgebaut wird und anschließend mit der Desorptionsanlage ein Volumenstrom in dem Behälter erzeugt wird.

Gemäß einer weiteren Ausführungsform des Verfahrens werden der Sterilisationsschritt und der Desorptionsschritt in derselben Anlage zeitlich direkt aufeinanderfolgend durchgeführt.

Zur Desorption wird der Wasserdampf insbesondere durch das Sorbat geleitet, sodass eine stetige Wasserdampfströmung durch das Sorbat eine Aufnahme der halogenierten Kohlenwasserstoffe in den Wasserdampf und somit deren Abtransport ermöglicht. Es bildet sich ein Gemisch aus Wasserdampf und halogenierten Kohlenwasserstoffen.

Dieses Gemisch wird gemäß einer Ausführungsform des Verfahrens von den mitgeführten Verunreinigungen befreit und auf eine Temperatur von unter 30°C gebracht. Es bildet sich ein zweiphasiges Flüssigkeitsgemisch, das Kondensat. Aus diesem Kondensat werden die halogenierten Kohlenwasserstoffe abgetrennt und weiterverarbeitet. Das Wasser kann in den Prozess zurückgeführt werden.

Gemäß einer weiteren Ausführungsform wird das Gemisch aus Wasserdampf und halogenierten Kohlenwasserstoffen zur Bildung eines zweiphasigen Flüssigkeitsgemisches (oder Kondensats) auf eine Temperatur unter 70°C, insbesondere unter 65°C, weiter insbesondere unter 60°C, weiter insbesondere unter 55°C, weiter insbesondere unter 50°C, weiter insbesondere unter 45°C, weiter insbesondere unter 40°C, weiter insbesondere unter 35°C, weiter insbesondere unter 30°C, weiter insbesondere unter 25°C, weiter insbesondere unter 20°C, weiter insbesondere unter 15°C, weiter insbesondere unter 10°C, weiter insbesondere unter 5°C, gebracht, wobei das Gemisch zuvor insbesondere von den mitgeführten Verunreinigungen befreit wird. Insbesondere wird dabei ein zweiphasiges Flüssigkeitsgemisch, das Kondensat, gebildet, wobei die halogenierten Kohlenwasserstoffe abgetrennt und weiterverarbeitet werden. Das Wasser kann dabei insbesondere in den Prozess zurückgeführt werden.

Die Temperatur, auf welche das Gemisch gebracht wird, um das Kondensat zu erzeugen, kann abhängig von der Siedetemperatur des zu desorbierenden halogenierten Kohlenwasserstoffes gewählt werden. Dabei muss die Temperatur unter der Siedetemperatur des entsprechenden Kohlenwasserstoffs liegen. Zusätzlich kann die Temperatur dahingehend optimiert werden, dass der Gasanteil über dem Desorbat-Wassergemisch, der sich typischerweise beim Absinken der schwereren Phase bildet, möglichst gering gehalten wird, um Verluste über die Gasphase zu minimieren. Neben der Siedetemperatur des entsprechenden halogenierten Kohlenwasserstoffes werden bei der Wahl der Temperatur, auf welche das Kondensat gebracht wird, auch Parameter der Prozessführung berücksichtigt. So können z.B. zu niedrige Temperaturen zum Einfrieren von Kühlern oder Leitungen führen, was den Prozess negativ beeinflusst. Die Siedetemperaturen einiger gängiger Inhalationsanästhetika sind z.B. wie folgt: Sevofluran: 58,5°C, Isofluran: 48,5°C, Desfluran: 22,5°C, Enfluran: 56,5°C, Halothan: 50,2°C.

Nach Desorption der halogenierten Kohlenwasserstoffe wird das Sorptionsmittel insbesondere gekühlt. Das nun sorptivfreie Sorptionsmittel kann für weitere Ad- /Desorptionszyklen bereitgestellt werden.

Gemäß einer weiteren Ausführungsform ist das Adsorbens, an dem die halogenierten Kohlenwasserstoffe adsorbiert werden, Aktivkohle, insbesondere hydrophobe Aktivkohle, und/oder Zeolith, insbesondere hydrophober Zeolith, insbesondere modifizierter hydrophober Zeolith. Das Adsorbens ist insbesondere porös und weist Poren, insbesondere im Bereich von Mikrometern und/oder Nanometern, auf. Es kann auch ein Adsorbentiengemisch verwendet werden.

Gemäß einer weiteren Ausführungsform der Erfindung ist das Adsorbens vor der Adsorption bzw. nach der Durchführung des gesamten Verfahrenszyklus und vor Einsatz zur Adsorption von Narkosemitteln durch einen Wassergehalt von ≤5% (w/w), insbesondere durch einen Wassergehalt von ≤2% (w/w) charakterisiert. Dieser Wasseranteil kann sich im Laufe des Verfahrens ändern.

Adsorbentien können sowohl hydrophil als auch hydrophob sein. Für das vorliegende Verfahren werden insbesondere hydrophobe Adsorbentien verwendet. Auch hydrophobe Adsorbentien können große Mengen Wasser aufnehmen. Eine Adsorption ist nur an den nicht durch Wasser besetzten Stellen des Adsorbens möglich. So soll das erfindungsgemäß verwendete Adsorbens insbesondere einen möglichst niedrigen Wassergehalt aufweisen, um möglichst viel halogenierte Kohlenwasserstoffe aufnehmen zu können. Nach der Desorption der halogenierten Kohlenwasserstoffe kann das Adsorbens z.B. einen Wassergehalt von über 30% (w/w) aufweisen. Nach Entfernung eines Großteils des Wassers kann das Adsorbens wiederverwendet werden.

Gemäß einer weiteren Ausführungsform enthält der im Desorptionsschritt verwendete Wasserdampf im Wesentlichen kein flüssiges Wasser, insbesondere weniger als 0,1 Gewichtsprozent flüssiges Wasser, weiter insbesondere kein flüssiges Wasser.

Man unterscheidet zwischen Nassdampf und Heißdampf. Heißdampf ist Dampf, der eine Temperatur oberhalb der Siedetemperatur des Wassers bei dem gegebenen Druck aufweist. Heißdampf enthält somit kein flüssiges Wasser mehr. Beim Nassdampf gibt es immer einen Anteil an Wassertröpfchen im Dampf.

Gemäß einer weiteren Ausführungsform ist der verwendete Wasserdampf Reinstdampf. Reinstdampf wird durch Verdampfen von vollentsalztem Wasser erhalten.

Gemäß einer weiteren Ausführungsform umfassen die halogenierten Kohlenwasserstoffe fluorhaltige Inhalationsanästhetika oder sind fluorhaltige Inhalationsanästhetika, insbesondere Sevofluran, Isofluran, Enfluran, Halothan, Desfluran oder Gemische daraus. Gemäß einer weiteren Ausführungsform wurde das die halogenierten Kohlenwasserstoffe umfassende Adsorbens durch Filterung von Atemluft aus der Behandlung von Patienten erhalten, die mit halogenierten Kohlenwasserstoffen narkotisiert wurden.

Im erfindungsgemäßen Verfahren werden der Sterilisationsschritt und der Desorptionsschritt in einem bis 0,4 MPa druckstabilen Desorptionsbehälter durchgeführt. Der Desorptionsbehälter weist insbesondere mindestens einen Dampfeingang und mindestens einen Dampfausgang auf. Über den Dampfeingang wird insbesondere Dampf in den Desorptionsbehälter eingespeist und verlässt den Desorptionsbehälter insbesondere über den Dampfausgang. Das die adsorbierten halogenierten Kohlenwasserstoffe umfassende Adsorbens ist insbesondere zwischen Dampfeingang und Dampfausgang so in dem Desorptionsbehälter angeordnet, dass in den Desorptionsbehälter durch den Dampfeingang eintretender Dampf das Adsorbens durchströmen muss, bevor es den Desorptionsbehälter durch den Dampfausgang verlässt. Jeder Punkt des die adsorbierten halogenierten Kohlenwasserstoffe umfassenden Adsorbens kommt so in Berührung mit dem Dampf. Beim Durchströmen des Adsorbens mit Wasserdampf bildet sich insbesondere ein Temperaturgradient vom Dampfeingang zum Dampfausgang aus. Der kälteste Punkt in dem Desorptionsbehälter befindet sich somit insbesondere am Dampfausgang. An dieser Stelle ist insbesondere ein Temperatursensor angebracht.

Gemäß einer Ausführungsform der Erfindung weist der Desorptionsbehälter ein Ventil auf, welches dem Dampfausgang nachgeschaltet ist, wobei das Ventil während des Sterilisationsschrittes geschlossen ist und während des Desorptionsschrittes geöffnet ist. Somit kann sich während des Sterilisationsschrittes der erforderliche Druck im Desorptionsbehälter einstellen. Während des Desorptionsschrittes ist das Ventil insbesondere geöffnet, sodass der sekundäre Volumenstrom den Desorptionsbehälter über den Dampfausgang verlassen kann.

Das die halogenierten Kohlenwasserstoffe umfassende Adsorbens kann gemäß einer Ausführungsform im Desorptionsbehälter in einem dem Desorptionsbehälter entnehmbaren Adsorbentienbehälter aufgenommen sein.

Der Desorptionsbehälter weist gemäß einer Ausführungsform eine umlaufende Wand und einen mit der umlaufenden Wand abschließenden Boden auf, wobei der Boden insbesondere ein gewölbter Boden, weiter insbesondere ein Klöpperboden nach DIN 28011 ist. Der Boden bildet insbesondere durch seine Wölbung einen Hohlraum unterhalb des in den Desorptionsbehälter eingebrachten Adsorbentienbehälters aus, und ist zur Aufnahme eines Kondensats eingerichtet. Das Kondensat umfasst insbesondere kondensierte Gase. Das Kondensat kann insbesondere sowohl Wasserdampf als auch die halogenierten Kohlenwasserstoffe umfassen. Der Boden ist nicht zwingend als Klöpperboden ausgebildet, aber für das erfindungsgemäße Verfahren von Vorteil, da somit das Desorbat-Wasser-Gemisch ohne Restverbleib über das Ausgangsventil weitergeleitet werden kann.

Der Boden ist gemäß einer Ausführungsform so gestaltet, dass er die maximal mögliche Menge an Kondensat unter Betriebsbedingungen aufnehmen kann. Die Menge aufgenommenen Kondensats hängt von der Temperaturdifferenz zum Desorptionsbehälter ab. In bestimmten Ausführungsformen ist vorgesehen, den Boden des Behälters noch vorzuheizen, um die Menge an Kondensat möglichst niedrig zu halten. Eine Restmenge an Kondensat ist in den meisten Betriebszuständen schwer zu vermeiden, da eine einmal gebildete Kondensatmenge durch die kleine Wärmeübertragungsfläche nicht mehr durch den nachfolgenden Dampf vollständig in die Gasphase überführt werden kann.

Gemäß einer weiteren Ausführungsform weist der Boden des Desorptionsbehälters einen ersten Mantel und einen zweiten Mantel auf, wobei zwischen dem erste Mantel und dem zweiten Mantel ein Zwischenraum ausgebildet ist, und wobei insbesondere während des Sterilisationsschrittes Dampf in den Zwischenraum eingeleitet wird, so dass der Boden geheizt wird.

Die in dem Desorptionsbehälter herrschende Temperatur wird insbesondere durch einen Temperatursensor gemessen und gesteuert. Der Temperatursensor befindet sich insbesondere unterhalb des in den Desorptionsbehälter eingebrachten Adsorbentienbehälters, weiter insbesondere zwischen dem Dampfausgang und dem in den Desorptionsbehälter eingebrachten Adsorbentienbehälter, insbesondere unmittelbar unterhalb des in den Desorptionsbehälter eingebrachten Adsorbentienbehälters.

Der Temperatursensor befindet sich gemäß einer Ausführungsform unmittelbar an der Unterseite des Adsorbentienbehälters. Dabei ist der Temperatursensor insbesondere so angeordnet, dass das Sensorelement sich genau unterhalb des Bodens des Adsorbentienbehälters befindet. Der Temperatursensor misst so immer die Temperatur der Dampfphase und nicht die des Kondensats, welches sich im Bereich des Bodens des Desorptionsbehälters ansammeln kann.

Gemäß einer Ausführungsform ist der Temperatursensor dazu eingerichtet, das Verfahren zu steuern. Sobald während des Sterilisationsschrittes eine vorgegebene Sterilisationstemperatur erreicht ist, wird eine eingestellte Sterilisationszeit gemessen. Nach Ablauf der Sterilisationszeit wird der Sterilisationsschritt beendet. Dies kann durch Öffnen des Ventils und somit durch Einleiten des Desorptionsschrittes erfolgen.

Gemäß einer weiteren Ausführungsform ist ein Steuergerät zur Steuerung des Verfahrens vorgesehen, wobei das Steuergerät dazu ausgebildet ist, das Verfahren anhand der von dem Temperatursensor gemessenen Temperatur, insbesondere der Dampfphase, zu steuern.

Gemäß einer Ausführungsform dient der Temperatursensor der Überwachung und der Steuerung des erfindungsgemäßen Verfahrens, aber nur soweit, dass der Sterilisationsprozess bei Unterschreiten der eingestellten Temperatur neu gestartet wird. Eine Verbindung mit dem Dampferzeuger im Prozess ist dabei insbesondere nicht gegeben. In dieser Ausführungsform wird der Prozess selbst insbesondere nur druckseitig über den eingestellten Betriebsdruck gefahren.

Gemäß einer weiteren Ausführungsform dient der Temperatursensor der Überwachung und der Steuerung des erfindungsgemäßen Verfahrens durch direkte Steuerung des Dampferzeugers und ggf. der angeschlossenen Ventile.

Gemäß einer Ausführungsform umfasst der Desorptionsbehälter zwei übereinander angeordnete Adsorbentienbehälter. Die Adsorbentienbehälter werden dabei insbesondere durch zwei jeweils über oder vor den Adsorbentienbehältern angeordnete Dampfeingänge mit Dampf beaufschlagt. Durch die Aufnahme von zwei Adsorbentienbehältern mit jeweils einem eigenen Dampfeingang verringert sich der Temperaturgradient innerhalb des Adsorbens. Eine Sterilisation ist somit auch bei einer geringeren Dampftemperatur, im Vergleich zu einer Vorrichtung mit nur einem Dampfeingang, an jeder Stelle des Adsorbens erfolgreich. Selbstverständlich kann der Desorptionsbehälter in analoger Weise auch drei oder mehr übereinander angeordnete Adsorbentienbehälter umfassen.

Der Adsorbentienbehälter umfasst gemäß einer weiteren Ausführungsform eine umlaufende Wand, einen mit der umlaufenden Wand abschließenden Boden und/oder einen Deckel. Gemäß einer weiteren Ausführungsform umfasst der Boden und/oder der Deckel ein gasdurchlässiges Filtergewebe, oder besteht daraus.

Das Filtergewebe weist gemäß einer Ausführungsform Poren im Bereich von 10-100 µm auf, weiter insbesondere in einem Bereich von 20-50 µm, insbesondere von 40 µm.

Gemäß einer weiteren Ausführungsform weist das Filtergewebe Poren einer Porengröße im Bereich von 10 nm bis 100 µm, insbesondere 100 nm bis 100 µm, weiter insbesondere 1 µm bis 100 µm, auf, wobei die Poren insbesondere unterschiedlich groß sein können.

Der/die Adsorbentienbehälter können in den Desorptionsbehälter eingeführt werden.

Die Wand des Adsorbentienbehälters kontaktiert die Wand des Desorptionsbehälters insbesondere nicht, wodurch sich ein Zwischenraum zwischen Desorptionsbehälterwand und Adsorbentienbehälterwand ausbildet.

Zur Vermeidung eines Dampfstroms im Zwischenraum zwischen Adsorbentienbehälter und Desorptionsbehälter ist der Deckel des Adsorbentienbehälters gemäß einer Ausführungsform mit einer Dichtung versehen, so dass der Dampf den Adsorbentienbehälter passieren muss. Diese Dichtung ist in bestimmten Ausführungsformen als aufblasbare Profildichtung ausgeführt.

Gemäß einer weiteren Ausführungsform ist eine, insbesondere umlaufende, Dichtung, zwischen dem Desorptionsbehälter und dem Adsorbentienbehälter (bzw. jedem Adsorbentienbehälter im Fall mehrerer Adsorbentienbehälter), vorgesehen, die den Zwischenraum zwischen dem Desorptionsbehälter und dem Adsorbentienbehälter, insbesondere gasdicht, verschließt. Insbesondere ist die Dichtung auf Höhe eines oberen Randes des Adsorbentienbehälters (bzw. des jeweiligen Adsorbentienbehälters) angeordnet, wenn der (jeweilige) Adsorbentienbehälter in den Desorptionsbehälter eingesetzt ist. Insbesondere wird die Dichtung mittels eines Überdrucks (z.B. eines inerten Gases, z.B. von bis zu 0,12 MPa über dem im Desorptionsbehälter herrschenden Prozessdruck) verformt, so dass die Dichtung den Zwischenraum, insbesondere gasdicht, verschließt (insbesondere wobei die Dichtung an eine Außenwandung des Adsorbentienbehälters oder an eine Innenwandung des Desorptionsbehälters gepresst wird). Gemäß einer Ausführungsform wird die Dichtung nur während des Desorptionsschrittes mittels des Überdrucks verformt. Während des Sterilisationsschrittes wird die Dichtung insbesondere nicht mit Überdruck beaufschlagt, so dass sich eine stabile Dampfatmosphäre (auch im Zwischenraum zwischen Adsorbentienbehälter und Desorptionsbehälter) ausbilden kann.

Ein weiterer Aspekt des Verfahrens ist die Rückgewinnung der halogenierten Kohlenwasserstoffe im Rückgewinnungsschritt. Hierzu wird der sekundäre Volumenstrom, der den Desorptionsbehälter über den Dampfausgang verlässt, gemäß einer weiteren Ausführungsform über eine Sammelleitung mittels eines, insbesondere spülbaren, Vorfilters und eines, insbesondere spülbaren, Nachfilters von den mitgeführten Verunreinigungen befreit. Gemäß einer Ausführungsform wird der sekundäre Volumenstrom durch nacheinander geschaltete Kühler, insbesondere drei solcher nachgeschalteten Kühler, auf eine Temperatur von unter 30°C gebracht.

Gemäß einer weiteren Ausführungsform wird der sekundäre Volumenstrom durch nacheinander geschaltete Kühler, insbesondere drei solcher nachgeschalteten Kühler, auf eine Temperatur von unter 70°C, insbesondere unter 65°C, weiter insbesondere unter 60°C, weiter insbesondere unter 55°C, weiter insbesondere unter 50°C, weiter insbesondere unter 45°C, weiter insbesondere unter 40°C, weiter insbesondere unter 35°C, weiter insbesondere unter 30°C, weiter insbesondere unter 25°C, weiter insbesondere unter 20°C, weiter insbesondere unter 15°C, weiter insbesondere unter 10°C, weiter insbesondere unter 5°C, gebracht.

Nach dem letzten Kühler wird das so gebildete Kondensat-Wasser-Gemisch insbesondere in einen Kondensatauffangbehälter überführt. Es bildet sich ein zweiphasiges Flüssigkeitsgemisch. Aus diesem Kondensat werden insbesondere die halogenierten Kohlenwasserstoffe abgetrennt und weiteverarbeitet. Das Wasser kann in den Verdampfungsvorgang zurückgeführt werden.

Ein weiterer Aspekt der vorliegenden Erfindung umfasst eine Vorrichtung zur Durchführung des dargestellten zweistufigen Verfahrens der Rückgewinnung von halogenierten Kohlenwasserstoffen. Diese Vorrichtung umfasst einen druckstabilen Desorptionsbehälter. Dieser Desorptionsbehälter umfasst
- einen zum Einlass von Wasserdampf in den Desorptionsbehälter eingerichteten Dampfeingang, insbesondere zum Verbinden mit einem Dampferzeuger,
- einen zum Abführen von Wasserdampf aus dem Desorptionsbehälter eingerichteten Dampfausgang mit einer in Richtung des Dampfaustritts hinter dem Dampfausgang angeordneten Ausleitung,
- ein Ventil, durch welches die Ausleitung verschließbar ist,
- einen Raum, insbesondere zwischen Dampfeingang und Dampfausgang, der zur Aufnahme eines Schüttguts, z.B. eines halogenierte Kohlenwasserstoffe umfassenden Adsorbens, ausgebildet ist.

Der Desorptionsbehälter ist bis 0,4 MPa druckstabil.

Der für das Verfahren erforderliche Dampf kann insbesondere durch einen Dampferzeuger erzeugt werden, welcher Teil der erfindungsgemäßen Vorrichtung ist. Alternativ dazu kann auch ein separater, externer Dampferzeuger an den Dampfeingang der Vorrichtung angeschlossen werden.

Die Bildung des Kondensats nach dem erfindungsgemäßen Verfahren kann insbesondere innerhalb des Desorptionsbehälters oder in einem separaten Kondensationsbereich bzw. Kondensationsbehälter erfolgen.

Das Ventil ist insbesondere druckstabil bis mindestens zu einem Druck von 0,4 MPa verschließbar. Das Ventil ist insbesondere dazu eingerichtet, den Druck innerhalb des Desorptionsbehälters zu steuern. Ist das Ventil geschlossen, ist es insbesondere dazu ausgebildet, einen Druckaufbau innerhalb des Desorptionsbehälters zu ermöglichen. Der Dampfausgang ist bei geschlossenem Ventil insbesondere versperrt. Ist das Ventil geöffnet, kann ein Volumenstrom den Desorptionsbehälter insbesondere über den Dampfausgang verlassen.

Gemäß einer Ausführungsform der erfindungsgemäßen Vorrichtung umfasst der Desorptionsbehälter mindestens einen zur Aufnahme des Schüttguts vorgesehenen entnehmbaren Adsorbentienbehälter und einen Temperatursensor, welcher auf der oder einer dem Dampfausgang zugewandten Seite und damit auf der dem Dampfeingang abgewandten Seite des Adsorbentienbehälters angeordnet ist. Der Temperatursensor ist insbesondere unmittelbar unterhalb des Adsorbentienbehälters angeordnet. Dabei bildet der Adsorbentienbehälter insbesondere zumindest einen Teil des Raumes, insbesondere den gesamten Raum, der zur Aufnahme des Schüttguts ausgebildet ist. Im Fall mehrerer Adsorbentienbehälter bilden die mehreren Adsorbentienbehälter insbesondere gemeinsam den besagten Raum zur Aufnahme des Schüttguts aus.

Gemäß einer Ausführungsform der Vorrichtung ist der Temperatursensor mit einer Steuereinrichtung verbunden, welche dazu ausgebildet ist, das Ventil nach Erreichen einer Zieltemperatur und/oder nach Ablauf einer vorgewählten Zeitspanne, insbesondere von 10 min bis 60 min, während derer die Zieltemperatur gehalten wird, zu öffnen. Die Steuereinrichtung kann sich außerhalb des Desorptionsbehälters befinden.

Gemäß einer weiteren Ausführungsform weist der Desorptionsbehälter einen gewölbten Boden, insbesondere einen Klöpperboden nach DIN 28011, auf. Der Boden ist insbesondere dazu eingerichtet, während des Sterilisationsschrittes kondensierte Anteile aufzunehmen.

Gemäß einer weiteren Ausführungsform ist der Desorptionsbehälter zur Aufnahme zweier übereinander angeordneter Adsorbentienbehälter eingerichtet, und mit zwei jeweils oberhalb der Adsorbentienbehälter angeordneten, Dampfeingängen ausgestaltet.

Gemäß einer weiteren Ausführungsform weist die Vorrichtung einen Dampferzeuger auf, der zur Erzeugung, insbesondere trockenen, Wasserdampfes eingerichtet ist, wobei der Dampferzeuger mit mindestens einem Dampfeingang in fluidischer Kommunikation steht bzw. verbunden ist.

Gemäß einer weiteren Ausführungsform weist die Vorrichtung eine dem Dampferzeuger nachgeschaltete Trocknungseinrichtung auf, wobei die Trocknungseinrichtung dazu ausgebildet ist, einen Heißdampfzustand unter den gegebenen Bedingungen herzustellen. Die Trocknungseinrichtung kann eine Tröpfchenabscheidekolonne sein.

Der Dampferzeuger weist insbesondere einen unteren und einen oberen Schaltpunkt zur Füllstandskontrolle auf. Der Wasserstand kann, insbesondere mittels der Füllstandskontrolle, kontrolliert werden, damit der Dampferzeuger nicht trocken läuft bzw. nicht überläuft und somit Dampf in gewünschter Qualität bereitgestellt werden kann.

Es kann vorgesehen sein, dass bei Unterschreiten eines vorbestimmten Füllstands der Dampferzeuger bzw. insbesondere die einzeln geschalteten Patronen a 5 KW automatisch abgeschaltet wird, um das Trockenlaufen und Durchglühen zu verhindern.

Gemäß einer weiteren Ausführungsform weist die Vorrichtung mehrere Desorptionsbehälter auf, wobei der Dampferzeuger mit jeweiligen Dampfeingängen der mehreren Desorptionsbehälter verbunden ist. In einer Ausführungsform ist der Dampferzeuger mit vier Desorptionsbehältern verbunden. Die maximale Produktionsleistung Reinstdampf liegt in dieser Ausführungsform insbesondere bei 60 kg/h, 0,2 MPa (1 Atmosphäre Überdruck). Diese wird insbesondere im Parallelanfahrbetrieb von vier Desorptionsbehältern voll genutzt. Im weiteren Betrieb liegt diese dann insbesondere bei annähernd einem Drittel bis der Hälfte davon.

Gemäß einer weiteren Ausführungsform ist die Vorrichtung dazu eingerichtet, die Desorptionsbehälter in unterschiedlicher Anzahl mit einem Zeitverzug von 1 min bis 2 min parallel zu fahren.

Gemäß einer weiteren Ausführungsform ist der Dampferzeuger dazu eingerichtet, aus vollentsalztem Wasser Reinstdampf herzustellen.

Das vollentsalzte Wasser wird gemäß einer Ausführungsform durch eine Wasseraufbereitungsvorrichtung bereitgestellt, die dem Dampferzeuger vorgeschaltet ist. Diese umfasst insbesondere eine Stadtwassereinspeisung, insbesondere eine Enthärtungsanlage und insbesondere nachfolgend ein Umkehrosmosemodul. Das aufbereitete Wasser wird gemäß einer Ausführungsform in einem mit dem Dampferzeuger verbundenen Wasserspeicher gespeichert. Zwischen der Stadtwassereinspeisung und der Enthärtungsanlage ist insbesondere ein Vorfilter eingebaut.

Der Vorfilter weist gemäß einer Ausführungsform Poren mit einer Größe von 50 µm bis maximal 100 µm auf.

Gemäß einer weiteren Ausführungsform weist der Vorfilter Poren einer Porengröße im Bereich von 10 nm bis 100 µm, insbesondere 100 nm bis 100 µm, weiter insbesondere 1 µm bis 100 µm, auf, wobei insbesondere die Poren unterschiedlich groß sein können.

Zwischen der Enthärtungsanlage und dem Umkehrosmosemodul ist insbesondere ein Feinfilter angeordnet.

Die Poren des Feinfilters sind gemäß einer Ausführungsform 2 µm bis 8 µm groß, wobei die Poren unterschiedlich groß sein können. Die durchschnittliche Größe beträgt in bestimmten Ausführungsformen 5 µm.

Gemäß einer weiteren Ausführungsform weist der Feinfilter Poren einer Porengröße im Bereich von 10 nm bis 8 µm, insbesondere 100 nm bis 8 µm, weiter insbesondere 1 µm bis 8 µm, auf, wobei insbesondere die Poren unterschiedlich groß sein können.

Gemäß einer weiteren Ausführungsform weist die Vorrichtung einen Kondensatbereich auf, wobei der Desorptionsbehälter mit dem Kondensatbereich verbunden ist, und wobei der Kondensatbereich dazu eingerichtet ist, den sekundären Volumenstrom zu kühlen und daraus ein Kondensat zu erzeugen. Zwischen Desorptionsbehälter und Kondensatbereich ist insbesondere ein Filter angeordnet. Dieser Filter ist insbesondere dazu eingerichtet, alle im sekundären Volumenstrom mitgeführten Partikel, die sich beim Durchströmen des Adsorbens davon lösen, herauszufiltern. Dieser Filter ist insbesondere spülbar. Es ist möglich, mehrere dieser Filter hintereinander anzuordnen.

In einer Ausführungsform sind zwischen Desorptionsbehälter und Kondensatbereich ein, insbesondere spülbarer, Vorfilter und ein, insbesondere spülbarer, Nachfilter angeordnet, wobei der Vorfilter dem Nachfilter vorgeschaltet ist.

Der Vorfilter weist gemäß einer Ausführungsform eine Porengröße von 15 µm bis 30 µm, insbesondere von ca. 25 µm auf.

Gemäß einer weiteren Ausführungsform weist der Vorfilter Poren einer Porengröße im Bereich von 10 nm bis 30 µm, insbesondere 100 nm bis 30 µm, weiter insbesondere 1 µm bis 30 µm, auf, wobei insbesondere die Poren unterschiedlich groß sein können.

Der Nachfilter weist gemäß einer Ausführungsform eine Porengröße von 1 µm bis 5 µm, insbesondere von ca. 5 µm auf.

Gemäß einer weiteren Ausführungsform weist der Nachfilter Poren einer Porengröße im Bereich von 10 nm bis 5 µm, insbesondere 100 nm bis 5 µm, weiter insbesondere 1 µm bis 5 µm, auf, wobei insbesondere die Poren unterschiedlich groß sein können.

Gemäß einer weiteren Ausführungsform umfasst der Kondensatbereich einen Vorkühler, einen Zwischenkühler sowie einen Nachkühler, die nacheinander angeordnet sind. Der Vorkühler- und der Zwischenkühler werden gemäß einer Ausführungsform mit (insbesondere ungekühltem) Stadtwasser betrieben oder sind mit (insbesondere ungekühltem) Stadtwasser betreibbar. Der Nachkühler wird insbesondere mit gekühltem Stadtwasser betrieben oder ist mit gekühltem Stadtwasser betreibbar. Zwischen dem Zwischenkühler und dem Nachkühler ist insbesondere ein Feinfilter angeordnet.

Alternativ dazu können auch alle Kühler, d.h. der Vorkühler, der Zwischenkühler und der Nachkühler mit gekühltem Stadtwasser betrieben werden, das sich entsprechend der Prozessführung in der Temperatur unterscheiden kann.

Der Feinfilter weist gemäß einer Ausführungsform eine Porengröße von 1 µm bis 5 µm, insbesondere von ca. 5 µm auf.

Gemäß einer weiteren Ausführungsform weist der Feinfilter Poren einer Porengröße im Bereich von 10 nm bis 5 µm, insbesondere 100 nm bis 5 µm, weiter insbesondere 1 µm bis 5 µm, auf, wobei insbesondere die Poren unterschiedlich groß sein können.

Gemäß einer weiteren Ausführungsform weist die Vorrichtung ein Filterspülsystem auf, wobei das Filterspülsystem dazu eingerichtet ist, den Vorfilter, den Zwischenfilter, den Nachfilter und/oder den Feinfilter, insbesondere automatisch, zu spülen. Gemäß einer weiteren Ausführungsform ist das Filterspülsystem dazu ausgebildet, den Vorfilter, den Zwischenfilter, den Nachfilter und/oder den Feinfilter mit unterschiedlichen Volumina einer Spülflüssigkeit, insbesondere Wasser (z.B. vollentsalztes Wasser, VE-Wasser) zu spülen. Gemäß einer weiteren Ausführungsform ist das Filterspülsystem dazu ausgebildet, den Vorfilter, den Zwischenfilter, den Nachfilter und/oder den Feinfilter zeitversetzt zu spülen. Das Filterspülsystem ist insbesondere mit einem Speicherbehälter zum Speichern der Spülflüssigkeit verbunden oder weist einen Speicherbehälter zum Speichern der Spülflüssigkeit auf. Insbesondere weist das Filterspülsystem eine Pumpe zum Pumpen der Spülflüssigkeit aus dem Speicherbehälter durch den Vorfilter, den Zwischenfilter, den Nachfilter und/oder den Feinfilter auf. Die obere Grenze für das Gesamtvolumen zur Spülung der Filter berechnet sich insbesondere aus dem Volumen des Speicherbehälters abzüglich einer Restfüllmenge, die so bemessen ist, dass die Pumpe nicht trockenläuft.

An den Kondensatbereich schließt sich gemäß einer weiteren Ausführungsform ein Auffangbereich an. Dieser umfasst insbesondere einen Kondensatauffangbehälter, einen Drucklufterzeuger sowie einen Pufferbehälter.

Der Kondensatauffangbehälter schließt sich insbesondere stromabwärts an den Nachkühler an. Dieser Kondensatauffangbehälter ist insbesondere dazu eingerichtet, das gekühlte Kondensat aufzufangen und in seine Phasen aufzutrennen.

Gemäß einer weiteren Ausführungsform weist die Vorrichtung einen Pufferbehälter auf, wobei die Vorrichtung dazu ausgebildet ist, bei Erreichen einer entsprechenden Füllhöhe die halogenierten Kohlenwasserstoffe, die aufgrund ihrer Dichte vom Wasser trennbar sind, mittels eines Auslasses in den Pufferbehälter zu überführen.

Gemäß einer weiteren Ausführungsform weist der die Vorrichtung eine im Zwischenraum zwischen dem Adsorbentienbehälter (bzw. jedem Adsorbentienbehälter) und dem Desorptionsbehälter angeordnete oder anordenbare, insbesondere umlaufende, Dichtung auf, die dazu ausgebildet ist, den Zwischenraum, insbesondere gasdicht, zu verschließen, wenn der Adsorbentienbehälter in den Desorptionsbehälter eingeführt ist und somit insbesondere einen Dampfstrom im Zwischenraum zwischen dem Adsorbentienbehälter und dem Desorptionsbehälter zu verhindern.

Gemäß einer weiteren Ausführungsform ist die Vorrichtung dazu ausgebildet, die Dichtung mittels eines Überdrucks (z.B. eines inerten Gases, z.B. von bis zu 0,12 MPa über dem im Desorptionsbehälter herrschenden Prozessdruck) zu verformen so dass die Dichtung den Zwischenraum, insbesondere gasdicht, verschließt (insbesondere wobei die Dichtung mittels des Überdrucks an eine Außenwandung des Adsorbentienbehälters oder an eine Innenwandung des Desorptionsbehälters pressbar ist).

Gemäß einer Ausführungsform ist die Vorrichtung dazu ausgebildet, die Dichtung nur während des Desorptionsschrittes mittels des Überdrucks zu verformen.

Gemäß einer weiteren Ausführungsform weist ein Deckel des Adsorbentienbehälters die Dichtung auf.

Gemäß einer weiteren Ausführungsform ist die Dichtung als aufblasbare Profildichtung ausgebildet.

Gemäß einer weiteren Ausführungsform weist der Desorptionsbehälter einen Boden auf, wobei der Boden einen ersten Mantel und einen zweiten Mantel aufweist, wobei zwischen dem erste Mantel und dem zweiten Mantel ein Zwischenraum ausgebildet ist, und wobei insbesondere die Vorrichtung dazu ausgebildet ist, während des Sterilisationsschrittes Dampf in den Zwischenraum einzuleiten, so dass der Boden heizbar ist.

### Figurenbeschreibung

Fig. 1 zeigt einen Desorptionsbehälter mit zwei entnehmbaren Adsorbentienbehältern.
Fig. 2 zeigt einen Adsorbentienbehälter.
Fig. 3 stellt einen schematischen Aufbau der Rückgewinnungsanlage dar.

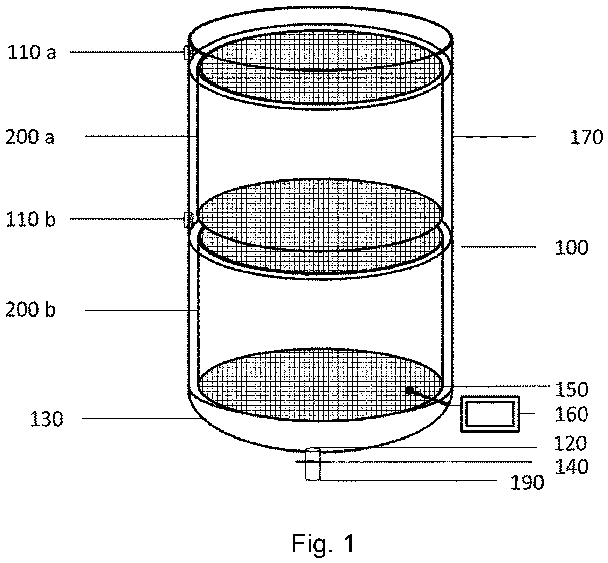

In Fig. 1 ist ein Desorptionsbehälter 100 dargestellt, der eine umlaufende Wand 170 und einen mit der umlaufenden Wand 170 abschließenden Boden 130 umfasst, zwei Dampfeingänge 110a, 110b und einen am Boden des Desorptionsbehälters 130 angeordneten Dampfausgang 120 aufweist. Am Dampfausgang ist ein Ventil 140 angeordnet. Dieses Ventil 140 ist eingerichtet, die stromabwärts an den Dampfausgang 120 anschließende Ausleitung 190 zu öffnen oder zu verschließen. In dem Desorptionsbehälter 100 sind zwei entnehmbare Adsorbentienbehälter 200a, 200b übereinander angeordnet. Die Dampfeingänge 110a, 100b sind oberhalb der entnehmbaren Adsorbentienbehälter 200a, 200b angeordnet. Zwischen dem Dampfausgang 120 und dem unteren Adsorbentienbehälter 200b ist unmittelbar unterhalb des unteren Adsorbentienbehälters 200b ein Temperatursensor 150 angeordnet. Dieser ist mit einer Steuereinrichtung 160 verbunden.

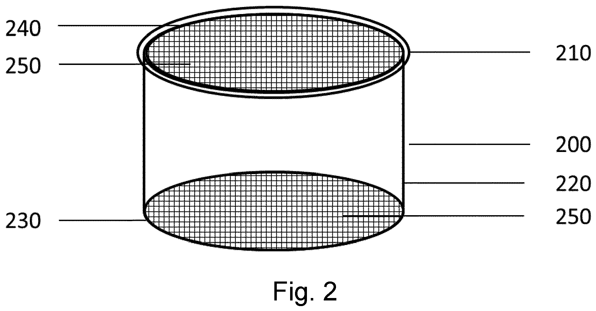

In Fig. 2 ist ein Adsorbentienbehälter 200 dargestellt. Dieser weist eine umlaufende Wand 220, damit abschließend einen Boden 230 und einen Deckel 240 auf. Der Boden 230 und der Deckel 240 des Adsorbentienbehälters sind mit einem Filtergewebe 250 ausgestaltet. Am Deckel 240 des Adsorbentienbehälters 200 ist eine Dichtung 210 angeordnet, die dazu eingerichtet ist, den Zwischenraum zwischen der Wand des Desorptionsbehälters 170 und des Adsorbentienbehälters 220 gasdicht zu verschließen, wenn der Adsorbentienbehälter in den Desorptionsbehälter eingeführt ist.

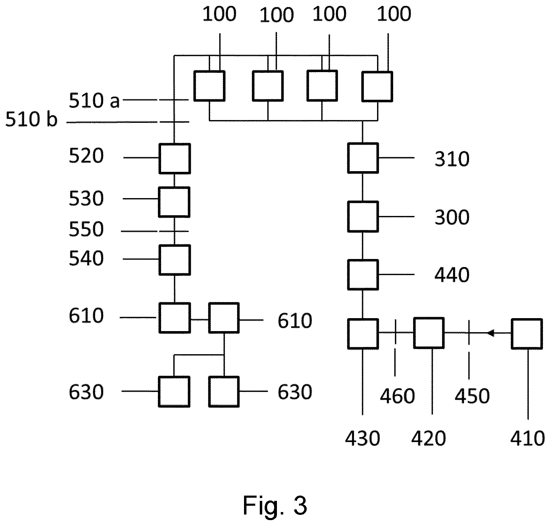

In Fig. 3 ist die Rückgewinnungsanlage schematisch dargestellt. In den Desorptionsbehältern 100 findet der Sterilisations- und Desorptionsvorgang statt. Hierzu benötigter trockener Reinstdampf wird im Dampferzeuger 300 mit nachgeschalteter Trocknungseinrichtung 310 bereitgestellt. Die Trocknungseinrichtung 310 kann eine Tröpfchenabscheidekolonne sein. Der Reinstdampf wird aus entsalztem Wasser hergestellt. Das entsalzte Wasser wird in einer dem Dampferzeuger 300 vorgeschalteten Reinigungseinrichtung bereitgestellt, welche dazu eingerichtet ist, Wasser aus einer Stadtwassereinspeisung 410 in einem ersten Schritt mittels einer Enthärtungsanlage 420 und in einem zweiten Schritt mittels eines Umkehrosmosemoduls 430 aufzureinigen. Zwischen Stadtwassereinspeisung 410 und Enthärtungsanlage 420 ist ein Vorfilter 450 angeordnet. Zwischen Enthärtungsanlage 420 und Umkehrosmosemodul 430 ist ein Feinfilter 460 angeordnet. Dem Umkehrosmosemodul 430 nachgeschaltet ist ein Wasserspeicher 440, der dazu eingerichtet ist, das aufgereinigte Stadtwasser für die Verwendung im Dampferzeuger 300 zu speichern. Die Desorptionsbehälter 100 sind zwischen dem Dampferzeuger 300 und einem Kondensatbereich angeordnet. Der Kondensatbereich ist dazu eingerichtet, das den Desorptionsbehälter 100 im Desorptionsschritt verlassende Gasgemisch zur Bildung eines Kondensats zu kühlen. Der Kondensatbereich umfasst einen Vorkühler 520, einen Zwischenkühler 530 sowie einen Nachkühler 540. Dem Vorkühler 520 vorgeschaltet sind ein Vorfilter (510a) und ein Nachfilter 510b. Zwischen Zwischenkühler 530 und Nachkühler 540 ist ein Feinfilter 550 angeordnet.

An den Kondensatbereich schließt sich ein Auffangbereich an. Ein oder mehrere miteinander verbundene Kondensatauffangbehälter 610 sind dem Nachkühler 540 nachgeschaltet und sind dazu eingerichtet, das den Nachkühler 540 passierende Kondensat aufzufangen und in seine Phasen aufzutrennen.

An den Kondensatauffangbehälter 610 können sich ein oder mehrere Pufferbehälter 630 anschließen, die dazu eingerichtet sind, das in seine Phasen getrennte Kondensat aufzunehmen. Alternativ dazu kann auch nur ein einziger Kondensatauffangbehälter 610 (ohne zusätzliche Pufferbehälter 630) vorgesehen sein, der das gesamte Kondensat eines Desorptionsschrittes (insbesondere zuzüglich einer bestimmten Wassermenge, die für das Abtrennen des Desorbats notwendig ist) aufnehmen kann.

Die Abführung des Desorbats aus dem Desorptionsbehälter 100 kann insbesondere mittels Nutzung der potentiellen Energie des Desorbats oder z.B. durch eine Beaufschlagung mit einem inerten Gas (z.B. Stickstoff) erfolgen.

### Beschreibung beispielhafter Ausführungsformen

### Beispiel 1

Die Einbringung des Heißdampfes ist in zwei Phasen unterteilt. In der ersten Phase strömt der Heißdampf bei geschlossenen Ausgangsventil von dem Dampferzeuger in den Desorptionsbehälter bis ein Druck von größer/gleich 0,2 MPa erreicht wird. Dieser Druck und die damit korrespondierende Temperatur werden über einen Zeitraum von 30 min gehalten. In dieser Zeit herrschen autoklavenähnliche Verhältnisse im Desorptionsbehälter, die eine vollständige Inaktivierung aller im Sorbat befindlichen Pathogene (Bakterien, Mikroplasmen, Pilze, Viren, Viroide, Prionen, und/ oder Parasiten). gewährleisten. Nach dieser Inaktivierungsphase schließt sich die Desorptionsphase über einen Zeitraum von 120 min an, bei der bei geöffneten Ausgangsventil eine stetige Heißdampfströmung durch den Desorptionsbehälter eine Aufnahme und Abtransport der aus dem Sorbat ausgetriebenen halogenierten Kohlenwasserstoffe ermöglicht. Dieses Kondensat-Dampf-Gemisch wird über eine Sammelleitung mittels eines spülbaren Vorfilters (Edelstahlfilter, 25 Mikrometer) und einem spülbaren Nachfilter (Edelstahl, 5 Mikrometer) von den mitgeführten Verunreinigungen befreit und durch drei nacheinander geschaltete Kühler auf eine Temperatur von unter 30 Grad gebracht. Abhängig von dem zu desorbierenden halogenhaltigen Kohlenwasserstoff, insbesondere Inhalationsanästhetikum, kann diese Temperaturdifferenz wie oben beschrieben angepasst werden. Nach dem letzten Kühler wird das Kondensat-Wasser-Gemisch in einen Kondensatauffangbehälter überführt.

### Beispiel 2

**Tabelle 1: Übersicht über die Prozessdaten verschiedener Sterilisationszyklen.**

| **Versuch** | **Eingabe T/°C (+15 K)** | **Tₘᵢₙ/ °C** | **Tₘₐₓ /° C** | **Druck/ bar** | **Zeit / min** | **Keime** |
|---|---|---|---|---|---|---|
| 1.1 | 121,11 (+15 K) | 80,6 | 127,56 | 2,4 | 28:46 min | An allen Positionen Keime |
| 1.2 | 121,11 (+15 K) | 116,62 | 130,93 | 2,5 | 28:50 min | An einer Position Keime |
| 1.3 | 121,11 (+15 K) | 124,59 | 141,44 | 2,4 | 28:10min | An allen Position keimfrei |
| 1.4 | 121,11 (+15 K) | 125,01 | 136,55 | 2,35 | 28:27 min | An 2 Positionen Keime |
| 2.1 | 121,11 (+15 K) | 126,30 | 134,57 | 2,44 | 32:53 min | An allen Positionen keimfrei |
| 2.2 | 121,11 (+15 K) | 100,40 | 130,07 | N/A | 34:49 min | An 2 Positionen Keime |
| 2.3 | 121,11(+15 K) | 64,03 | 128,16 | 2,48 | 30:15 min | An 6 Positionen Keime |
| 2.4 | 121,11 (+15 K) | 121,68 | 129,29 | 2,5 | 28:27 | An allen Positionen keimfrei |
| 2.5 | 121,11 (+15K) | 127,25 | 131,34 | 2,5 | 30:22 | An allen Positionen keimfrei |
| | | | | | | |
| 3.1 | 121,1 (+25K) | 134,08 | 144,57 | 3,0 | 33:24 | Keine Angaben |
| 3.2 | 121,1 (+25K) | 132,15 | 146,46 | 3,0 | 37:31 | Keine Angaben |
| 3.3 | 121,1 (+25K) | 131,0 | 143,96 | 3,0 | 36:44 | Keine Angaben |
| 3.4 | 121,11 (+25K) | 134,89 | 138,08 | 3,0 | 29:03 | Keine Angaben |

### Bezugszeichenliste

- 100: Desorptionsbehälter
- 110: Dampfeingang
- 110 a: erster Dampfeingang
- 110 b: zweiter Dampfeingang
- 120: Dampfausgang
- 130: Boden des Desorptionsbehälters
- 140: Ventil
- 150: Temperatursensor
- 160: Steuereinrichtung
- 170: Wand des Desorptionsbehälters
- 190: Ausleitung
- 200: Adsorbentienbehälter
- 210: Dichtung
- 220: Wand des Adsorbentienbehälters
- 230: Boden des Adsorbentienbehälters
- 240: Deckel des Adsorbentienbehälters
- 250: Filtergewebe
- 300: Dampferzeuger
- 310: Trocknungseinrichtung
- 410: Stadtwassereinspeisung
- 420: Enthärtungsanlage
- 430: Umkehrosmosemodul
- 440: Wasserspeicher
- 450: Vorfilter
- 460: Feinfilter
- 510: Filter
- 510 a: Vorfilter
- 510 b: Nachfilter
- 520: Vorkühler
- 530: Zwischenkühler
- 540: Nachkühler
- 550: Feinfilter
- 610: Kondensatauffangbehälter
- 620: Drucklufterzeuger
- 630: Pufferbehälter

## Patentansprüche

1. Verfahren zur Rückgewinnung von fluorhaltigen Inhalationsanästhetika,
wobei in einem Desorptionsschritt ein adsorbierte fluorhaltige Inhalationsanästhetika umfassendes Adsorbens von Wasserdampf durchströmt wird, wodurch ein fluorhaltige Inhalationsanästhetika enthaltender sekundärer Volumenstrom entsteht,
und wobei der sekundäre Volumenstrom durch Kühlung in ein fluorhaltige Inhalationsanästhetika und Wasser enthaltendes Kondensat überführt wird, aus welchem die fluorhaltigen Inhalationsanästhetika abgetrennt werden,
**dadurch gekennzeichnet, dass**
in einem dem Desorptionsschritt vorangehenden Sterilisationsschritt das adsorbierte fluorhaltige Inhalationsanästhetika umfassende Adsorbens
- für mindestens 10 min, insbesondere für 10 bis 60 min,
- bei einer Temperatur von mehr als 120°C, und
- bei einem Druck von 0,15 MPa bis 0,4 MPa,
in Kontakt mit Wasserdampf gebracht wird, wobei der Sterilisationsschritt und der Desorptionsschritt in derselben Anlage direkt aufeinanderfolgend durchgeführt werden;
und wobei
- der Sterilisationsschritt und der Desorptionsschritt in einem Desorptionsbehälter (100) durchgeführt werden,
- wobei der Desorptionsbehälter (100)
○ einen Dampfeingang (110) und einen Dampfausgang (120) aufweist, und
○ das Adsorbens zwischen Dampfeingang (110) und Dampfausgang (120) so in dem Desorptionsbehälter (100) angeordnet wird, dass in den Desorptionsbehälter (100) durch den Dampfeingang (110) eintretender Dampf das Adsorbens durchströmen muss, bevor es den Desorptionsbehälter (100) durch den Dampfausgang (120) verlässt, sowie
○ der Desorptionsbehälter (100) ein Ventil (140) aufweist, welches dem Dampfausgang (120) nachgeschaltet ist, wobei das Ventil (140) während des Sterilisationsschrittes geschlossen ist und während des Desorptionsschrittes geöffnet ist.

2. Das Verfahren gemäß Anspruch 1, wobei der Sterilisationsschritt bei einer Temperatur von 121 bis 150°C durchgeführt wird.

3. Das Verfahren gemäß Anspruch 1 oder 2, wobei der Sterilisationsschritt bei bei einem Druck von 0,15 bis 0,3 MPa durchgeführt wird.

4. Das Verfahren gemäß einem der vorstehenden Ansprüche, wobei das die adsorbierten fluorhaltigen Inhalationsanästhetika umfassende Adsorbens durch Filterung von Atemluft aus der Behandlung von Patienten erhalten wurde.

5. Das Verfahren gemäß einem der vorstehenden Ansprüche, wobei der Sterilisationsschritt
- für 20 bis 40 min, insbesondere für ungefähr 30 min
- bei einer Temperatur von 135 bis 145°C, und
- bei einem Druck von 0,24 bis 0,26 MPa
durchgeführt wird.

6. Das Verfahren gemäß einem der vorstehenden Ansprüche, wobei die in dem Desorptionsbehälter (100) herrschende Temperatur durch einen Temperatursensor (150) gemessen wird, und wobei der Temperatursensor (150) sich unterhalb des in den Desorptionsbehälter (100) eingebrachten Adsorbentienbehälters (200) befindet, insbesondere zwischen dem Dampfausgang (120) und dem in den Desorptionsbehälter (100) eingebrachten Adsorbentienbehälter (200), insbesondere unmittelbar unterhalb des in den Desorptionsbehälter (100) eingebrachten Adsorbentienbehälters (200).

7. Das Verfahren gemäß einem der vorstehenden Ansprüche, wobei der Desorptionsbehälter (100) zwei übereinander angeordnete Adsorbentienbehälter (200a, 200b) umfasst, und die Adsorbentienbehälter (200a, 200b) durch zwei jeweils über den Adsorbentienbehältern (200a, 200b) angeordnete Dampfeingänge (110a, 110b) mit Dampf beaufschlagt werden können.

8. Das Verfahren gemäß einem der vorstehenden Ansprüche, wobei der Adsorbentienbehälter (200) einen Boden (230) und/oder einen Deckel (240) aufweist, wobei der Boden (230) und/oder der Deckel (240) ein gasdurchlässiges Filtergewebe (250) umfassen oder daraus bestehen.

9. Eine Vorrichtung zur Durchführung eines Verfahrens gemäß einem der vorstehenden Ansprüche, umfassend
- einen bis 0,4 MPa druckstabilen Desorptionsbehälter (100),
- einen zum Einlass von Wasserdampf in den Desorptionsbehälter (100) eingerichteten Dampfeingang (110),
- einen zum Abführen von Wasserdampf aus dem Desorptionsbehälter (100) eingerichteten Dampfausgang (120) mit einer in Richtung des Dampfaustritts hinter dem Dampfausgang (120) angeordneten Ausleitung (190);
- ein Ventil (140), durch welches die Ausleitung (190) verschließbar ist,
- einen Raum, der zur Aufnahme eines Schüttguts ausgebildet ist und dieses enthält, wobei das Schüttgut ein fluorhaltige Inhalationsanästhetika umfassendes Adsorbens ist;
- wobei der Desorptionsbehälter (100) einen zur Aufnahme des Schüttguts vorgesehenen Adsorbentienbehälter (200), welcher den Raum zur Aufnahme des Schüttguts ausbildet, und einen Temperatursensor (150), welcher auf der dem Dampfausgang (120) zugewandten Seite des Adsorbentienbehälters (200) angeordnet ist, umfasst.

10. Die Vorrichtung gemäß Anspruch 9, wobei der Temperatursensor (150) mit einer Steuereinrichtung (160) verbunden ist, welche dazu ausgebildet ist, das Ventil (140) nach Erreichen einer Zieltemperatur und/oder nach Ablauf einer vorgewählten Zeitspanne von 10 bis 60 min, während derer die Zieltemperatur gehalten wird, zu öffnen.

11. Die Vorrichtung gemäß einem der Ansprüche 9 oder 10, wobei der Desorptionsbehälter zur Aufnahme zweier übereinander angeordneter Adsorbentienbehälter (200a, 200b) eingerichtet, und mit zwei jeweils oberhalb der Adsorbentienbehälter (200a, 200b) angeordneten, Dampfeingängen (110a, 110b) ausgestaltet ist.

12. Die Vorrichtung gemäß einem der Ansprüche 9 bis 11, ferner umfassend einen mit dem Desorptionsbehälter (100) verbundenen Kondensatbereich, der dazu eingerichtet ist, einen fluorhaltige Inhalationsanästhetika und Wasserdampf enthaltenden sekundären Volumenstrom zu kühlen, der in einem Desorptionsschritt durch Durchströmen des fluorhaltige Inhalationsanästhetika umfassenden Adsorbens mit dem Wasserdampf entsteht, und aus dem sekundären Volumenstrom ein Kondensat zu erzeugen.

13. Die Vorrichtung gemäß einem der Ansprüche 9 bis 12, aufweisend einen Dampferzeuger (300), der zur Erzeugung von Wasserdampf eingerichtet ist, wobei der Dampferzeuger (300) mit einem Dampfeingang (110) in fluidischer Kommunikation steht.

## Claims

1. Method for recovery of fluorine-containing inhalation anesthetics,
wherein, in a desorption step, an adsorbent comprising adsorbed fluorine-containing inhalation anesthetics is flowed through by water steam, whereby a secondary volume flow containing fluorine-containing inhalation anesthetics forms,
and wherein the secondary volume flow is transferred by cooling into a condensate containing fluorine-containing inhalation anesthetics and water, from which the fluorine-containing inhalation anesthetics are separated,
**characterized in that**
in a sterilization step preceding the desorption step, the adsorbent comprising adsorbed fluor-containing inhalation anesthetics
- for at least 10 min, particularly for 10 to 60 min,
- at a temperature of more than 120°C, and
- at a pressure of 0.15 MPa to 0.4 MPa,
is brought into contact with water steam, wherein the sterilization step and the desorption step are carried out in direct succession in the same plant;
and wherein
- the sterilization step and the desorption step are carried out in a desorption vessel (100),
- wherein the desorption vessel (100)
○ comprises a steam inlet (110) and a steam outlet (120), and
○ the adsorbent is arranged in the desorption vessel (100) between the steam inlet (110) and the steam outlet (120) such that steam entering the desorption vessel (100) through the steam inlet (110) must flow through the adsorbent before it leaves the desorption vessel (100) through the steam outlet (120), as well as
○ the desorption vessel (100) comprises a valve (140) which is subsequent to the steam outlet (120), wherein the valve (140) is closed during the sterilization step and is open during the desorption step.

2. The method according to claim 1, wherein the sterilization step is carried out at a temperature of 121 to 150°C.

3. The method according to claim 1 or 2, wherein the sterilization step is carried out at a pressure of 0.15 to 0.3 MPa.

4. The method according to any one of the preceding claims, wherein the adsorbent comprising adsorbed fluorine-containing inhalation anesthetics was obtained by filtering breathing air from the treatment of patients.

5. The method according to any one of the preceding claims, wherein the sterilization step is carried out
- for 20 to 40 min, particularly for about 30 min
- at a temperature of 135 to 145°C, and
- at a pressure of 0.24 to 0.26 MPa.

6. The method according to any one of the preceding claims, wherein the temperature prevailing in the desorption vessel (100) is measured by a temperature sensor (150), and wherein the temperature sensor (150) is located below the adsorbents vessel (200) introduced into the desorption vessel (100), in particular between the steam outlet (120) and the adsorbents vessel (200) introduced into the desorption vessel (100), in particular immediately below the adsorbents vessel (200) introduced into the desorption vessel (100).

7. The method according to one of the preceding claims, wherein the desorption vessel (100) comprises two adsorbents vessels (200a, 200b) arranged on top of each other, and the adsorbents vessels (200a, 200b) can be pressurized with steam through two steam inlets (110a, 110b) arranged respectively above the adsorbents vessels (200a, 200b).

8. The method according to any one of the preceding claims, wherein the adsorbents vessel (200) comprises a bottom (230) and/or a lid (240), wherein the bottom (230) and/or the lid (240) comprise or consist of a gas-permeable filter fabric (250).

9. A device for carrying out a method according to any one of the preceding claims, comprising
- an up to 0.4 MPa pressure-stable desorption vessel (100),
- a steam inlet (110) configured to introduce water steam into the desorption vessel (100),
- a steam outlet (120) configured to discharge water steam from the desorption vessel (100), with an outlet (190) arranged downstream of the steam outlet (120) in the direction of the steam outlet;
- a valve (140) through which the outlet (190) can be closed,
- a space formed to receive and contain a bulk material, wherein the bulk material comprises an adsorbent comprising fluorine-containing inhalation anesthetic;
- wherein the desorption vessel (100) comprises an adsorbents vessel (200) intended for receiving the bulk material, which forms the space for receiving the bulk material, and a temperature sensor (150), which is arranged on the side of the adsorbents vessel (200) facing the steam outlet (120).

10. The device according to claim 9, wherein the temperature sensor (150) is connected to a control device (160) which is designed to open the valve (140) after reaching a target temperature and/or after expiration of a preselected time period of 10 to 60 min, during which the target temperature is maintained.

11. The device according to one of claims 9 or 10, wherein the desorption vessel is arranged to receive two adsorbents vessels (200a, 200b) arranged on top of each other, and is designed with two steam inlets (110a, 110b) arranged respectively above the adsorbents vessels (200a, 200b).

12. The device according to any one of claims 9 to 11, further comprising a condensate section connected to the desorption vessel (100), which is configured to cool a secondary volume flow comprising fluorine-containing inhalation anesthetics and water steam, which is produced in a desorption step by flowing through the adsorbent comprising fluorine-containing inhalation anesthetics with the water steam, and to generate a condensate from the secondary volume flow.

13. The device according to any one of claims 9 to 12, comprising a steam generator (300) configured to generate water steam, wherein the steam generator (300) is in fluidic communication with a steam inlet (110).

## Revendications

1. Procédé de récupération d'anesthésiques par inhalation fluorés,
dans lequel un adsorbant comprenant des anesthésiques par inhalation fluorés adsorbés est parcouru par de la vapeur d'eau dans une étape de désorption, moyennant quoi un courant volumique secondaire contenant des anesthésiques par inhalation fluorés apparaît,
et dans lequel le courant volumique secondaire est transformé par refroidissement en un condensat contenant des anesthésiques par inhalation fluorés et de l'eau, duquel les anesthésiques par inhalation fluorés sont séparés,
**caractérisé en ce que**
l'adsorbant comprenant des anesthésiques par inhalation fluorés adsorbés est mis en contact avec de la vapeur d'eau dans une étape de stérilisation précédant l'étape de désorption
- pendant au moins 10 min, notamment pendant 10 à 60 min,
- à une température de plus de 120 °C, et
- à une pression de 0,15 MPa à 0,4 MPa,
dans lequel l'étape de stérilisation et l'étape de désorption sont réalisées de manière directement successive dans la même installation ;
et dans lequel
- l'étape de stérilisation et l'étape de désorption sont réalisées dans un récipient de désorption (100),
- dans lequel le récipient de désorption (100)
○ présente une entrée de vapeur (110) et une sortie de vapeur (120), et
○ l'adsorbant est disposé entre l'entrée de vapeur (110) et la sortie de vapeur (120) dans le récipient de désorption (100) de sorte que de la vapeur entrant dans le récipient de désorption (100) à travers l'entrée de vapeur (110) doit parcourir l'adsorbant avant qu'il ne quitte le récipient de désorption (100) à travers la sortie de vapeur (120), ainsi que
○ le récipient de désorption (100) présente une vanne (140) qui est montée en aval de la sortie de vapeur (120), dans lequel la vanne (140) est fermée pendant l'étape de stérilisation et est ouverte pendant l'étape de désorption.

2. Procédé selon la revendication 1, dans lequel l'étape de stérilisation est réalisée à une température de 121 à 150 °C.

3. Procédé selon la revendication 1 ou 2, dans lequel l'étape de stérilisation est réalisée à une pression de 0,15 à 0,3 MPa.

4. Procédé selon une des revendications précédentes, dans lequel l'adsorbant comprenant des anesthésiques par inhalation fluorés adsorbés a été obtenu par filtration d'air respiratoire provenant du traitement de patients.

5. Procédé selon une des revendications précédentes, dans lequel l'étape de stérilisation est réalisée
- pendant 20 à 40 min, notamment pendant environ 30 min,
- à une température de 135 à 145 °C, et
- à une pression de 0,24 à 0,26 MPa.

6. Procédé selon une des revendications précédentes, dans lequel la température régnant dans le récipient de désorption (100) est mesurée par un capteur de température (150), et dans lequel le capteur de température (150) se trouve en dessous du récipient d'adsorbants (200) introduit dans le récipient de désorption (100), notamment entre la sortie de vapeur (120) et le récipient d'adsorbants (200) introduit dans le récipient de désorption (100), notamment directement en dessous du récipient d'adsorbants (200) introduit dans le récipient de désorption (100).

7. Procédé selon une des revendications précédentes, dans lequel le récipient de désorption (100) comprend deux récipients d'adsorbants (200a, 200b) disposés l'un au-dessus de l'autre, et les récipients d'adsorbants (200a, 200b) peuvent être sollicités avec de la vapeur à travers deux entrées de vapeur (110a, 110b) disposées chacune au-dessus des récipients d'adsorbants (200a, 200b).

8. Procédé selon une des revendications précédentes, dans lequel le récipient d'adsorbants (200) présente un fond (230) et/ou un couvercle (240), dans lequel le fond (230) et/ou le couvercle (240) comprennent un tissu filtrant perméable au gaz (250) ou se composent de celui-ci.

9. Dispositif pour la réalisation d'un procédé selon une des revendications précédentes, comprenant
- un récipient de désorption (100) stable en pression jusqu'à 0,4 MPa,
- une entrée de vapeur (110) configurée pour l'entrée de vapeur d'eau dans le récipient de désorption (100),
- une sortie de vapeur (120) configurée pour la purge de vapeur d'eau du récipient de désorption (100) avec une diversion (190) disposée en direction de l'évacuation de vapeur derrière la sortie de vapeur (120) ;
- une vanne (140) par laquelle la diversion (190) peut être fermée,
- un espace qui est réalisé pour la réception d'un produit en vrac et contient celui-ci, dans lequel le produit en vrac est un adsorbant comprenant des anesthésiques par inhalation fluorés ;
- dans lequel le récipient de désorption (100) comprend un récipient d'adsorbants (200) prévu pour la réception du produit en vrac qui forme l'espace pour la réception du produit en vrac, et un capteur de température (150) qui est disposé sur le côté du récipient d'adsorbants (200) tourné vers la sortie de vapeur (120).

10. Dispositif selon la revendication 9, dans lequel le capteur de température (150) est connecté à un dispositif de commande (160) qui est réalisé pour ouvrir la vanne (140) après atteinte d'une température cible et/ou après écoulement d'un intervalle de temps présélectionné de 10 à 60 min pendant lequel la température cible est maintenue.

11. Dispositif selon une des revendications 9 ou 10, dans lequel le récipient de désorption est configuré pour la réception de deux récipients d'adsorbants (200a, 200b) disposés l'un au-dessus de l'autre, et structuré avec deux entrées de vapeur (110a, 110b) disposées chacune au-dessus des récipients d'adsorbants (200a, 200b).

12. Dispositif selon une des revendications 9 à 11, comprenant en outre une région de condensat connectée au récipient de désorption (100) qui est configurée pour refroidir un courant volumique secondaire contenant des anesthésiques par inhalation fluorés et de la vapeur d'eau qui apparaît dans une étape de désorption par parcours de l'adsorbant comprenant des anesthésiques par inhalation fluorés avec la vapeur d'eau, et pour générer un condensat à partir du courant volumique secondaire.

13. Dispositif selon une des revendications 9 à 12, présentant un générateur de vapeur (300) qui est configuré pour la génération de vapeur d'eau, dans lequel le générateur de vapeur (300) est en communication fluidique avec une entrée de vapeur (110).
